# EUROPEAN PATENT SPECIFICATION

(11) **EP 2 157 171 B1**
(45) Date of publication and mention of the grant of the patent: **25.02.2015**
(21) Application number: 08014921.4
(22) Date of filing: 22.08.2008
(51) Int. Cl.: C12N 5/00

(54) **Method for locally modifying the elasticity of a substrate designed for the growth of cells thereon**
Verfahren zur lokalen Modifizierung der Elastizität eines als Oberfläche zum Zellwachstum entwickelten Substrats
Procédé de modification locale de l'élasticité d'un substrat conçu pour la croissance de ses cellules

(43) Date of publication of application: 24.02.2010
(73) Proprietor: Universität Leipzig, 04109 Leipzig (DE)
(72) Inventor: Ehrlicher, Allen, 04317 Leipzig (DE); Käs, Josef, 04105 Leipzig (DE); Robitzky, Andrea, 68519 Viernheim (DE)
(74) Representative: Pfenning, Meinig & Partner GbR

(56) References cited:
- EP-A- 0 402 718
- EP-A- 0 841 361
- US-A- 5 137 800
- BURTON K. ET AL: "Traction forces of cytokinesis measured with optically modified elastic substrata" NATURE, NATURE PUBLISHING GROUP, LONDON, UK, vol. 385, no. 6615, 30 January 1997 (1997-01-30), pages 450-454, XP002513301 ISSN: 0028-0836
- WONG JOYCE Y. ET AL: "Directed movement of vascular smooth muscle cells on gradient-compliant hydrogels" LANGMUIR, ACS, WASHINGTON, DC, US, vol. 19, no. 5, 1 January 2003 (2003-01-01), pages 1908-1913, XP002513302 ISSN: 0743-7463 [retrieved on 2002-11-12]

## Description

The present invention deals with a method for locally modifying the elasticity of an elastic substrate, the substrate being designed for the growth of living cells thereon and/or therein, and with uses of such a method.

The present invention is based on the observation that the behavior of cells is dependent upon the elasticity of a substrate the cells are distributed in. In order to exploit this effect, there have been developed previous techniques of using UV lamps for setting up a desired elasticity in a cell substrate (see for example Burton and Taylor, Nature, 385, 1997, reducing the crosslinks in silicon rubbers and thus softening these rubbers in order to decrease the elasticity, or Wong et al., Langmuir, 19, 2003 in which photoinitializers were used with UV lamps to stiffen polyacrylamide gels).

With these prior art techniques, however, only large areas within the elastic material (substrate) to be modified can be altered with respect to the elasticity. Furthermore, the expenditure of time necessary in order to set up the desired elasticity is rather high. Beyond this, it is also difficult to control the lamps in such a way that the desired elasticity can be realized precisely.

Based on the above mentioned state of the art techniques, it is thus the object of the present invention to provide a method which is able to modify the elasticity of a substrate used for the growth of living cells thereon and/or therein precisely, with a low expenditure of time and with a high spatial resolution.

This object is solved by independent claim 1 of the present invention. Advantageous embodiments of the method according to the invention are described in the subsequent depending claims. Uses of the method according to the invention are described in claim 14.

The present invention is now further described: First in a more general way, then in a more specific way with a concrete example. The concrete method steps realized in said example (or described in the general description now following), however, do not have to be realized in exactly the described form, but can be realized within the scope of the appending claims also in other configurations or sequences of steps.

The basic idea of the present invention is to use a laser (e.g. a UV laser) adapted and arranged to realize a sufficient energy density in the substrate whose elasticity shall be modified and to focus its laser beam to a precisely defined location in or on the substrate. A sufficient energy density herein means that the energy locally introduced into the substrate material is high enough in order to modify the stiffness, elastic modulus and/or the elasticity of the elastic substrate, e.g. by breaking corresponding crosslinkers of the substrate material (i.e. reducing the number of crosslinkers per volume). The necessary energy density in order to accomplish this is dependent upon the specific substrate material and upon the strength of the desired elasticity modification. If therefore the locally introduced energy density is higher than a substrate material dependent threshold, the stiffness of the material is decreased. The introduced energy density in varied materials may have significantly different effects. For example, in a crosslinked polyacrylamide gel the energy may break crosslinks causing the gel to become less stiff, while in a protein based gel the energy may denature or otherwise change the protein causing changes which may include gel stiffening (i.e. the energy deposit increases the gel stiffness).

The local deposition of the energy in an arbitrary spatial area to be modified with respect to the local elasticity is realized by focusing the laser radiation onto the spatial area. Such a spatial area can for example be a dot-like area, a line-like area, a curve-like area or the like or any combination thereof in the substrate. If a plurality of such spatial areas are treated with energy deposits, a defined spatial pattern of locally varying elasticity can be realized (under the assumption that the respective local energy deposits are varied spatially). Thus, a path for cell guidance can be created inside the substrate (or also at the surface of the substrate). For example, this path for cell guidance can have a gradient-shaped variation of the elasticity: This means that for example along a straight line through the substrate of a cross-linked gel, the energy deposit is increased step by step so that the stiffness of the substrate material at the corresponding spatial areas is decreased step by step in this direction, while in a protein gel the stiffness may be increased.

An important aspect of the present invention is that the corresponding spatial areas can be modified with respect to the local elasticity, elastic modulus and/or stiffness before they are populated with living cells to be grown or distributed inside the substrate. It is therefore possible to realize a predetermined spatial pattern of modified areas inside a three-dimensional substrate (which can, preferably, be a gel, polymer, protein such as a collagen matrix or similar elastic materials which might be used with motile cells) with areas of larger elasticity and areas of smaller elasticity. If the spatial structure of said pattern is realized in an appropriate way, it is therefore possible to realize cell guidance paths inside or on the substrate which allow contacts to be formed between a first cell type and a second, same or different cell type: It is for example known that neurons prefer substrate materials with higher elasticity (softer materials) and that muscle cells prefer substrate materials with lower elasticity (stiffer materials). For example if the corresponding stiffer pattern areas are realized adjacent to areas of less stiffness, muscle cells can be distributed adjacent to neurons in order to allow a contact between those two cell types. This is only one example of many possible single cell or tissue interactions which can be controlled or guided by a specific spatially elastic patterned substrate. Therefore, cell cultures with a co-culturing of multiple cell types are possible with the present invention.

One reason that in one embodiment the spatial areas modified with respect to their elasticity are populated after the local energy deposition therein is that in general the energy deposit necessary in order to realize the desired elasticity is of an amount that may kill or harm the cells. It is possible, however, by optimizing certain experimental parameters such as radiation wavelength, duration, power, and proximity to cells to overcome this limitation and modify the substrate elasticity with cells present.

As is described in more detail later, to focus the laser radiation onto the predetermined spatial areas a microscope objective, preferably the objective of an inverted microscope (e.g. a conventionally inverted fluorescence microscope) can be used. The laser to introduce the energy can be a UV laser or other laser employed to realize a sufficient energy deposit. As an infrared laser, preferably a Ti:sapphire laser and/or an infrared laser with a wavelength between 750 nm and 1064 nm can be used. The laser can be a continuous wave laser or a pulsed laser, in the latter case preferably with a pulse frequency between 5 and 30 Hz depositing prefereably 100-500 µJ per pulse.

For the substrate used to grow living cells thereon and/or therein, preferably a gel, such as but not limited to polyacrylamide, agarose, fibrin and/or thrombin, or a collagen matrix can be used.

The cells used to populate the substrate can be any type which interact with a substrate, such as but not limited to eukaryotic cells, especially mammalian cells, such as human cells or animal cells, especially neurons and/or neuronal precursors and muscle cells. However, many other cell types can conceivably be used, such as prokaryotes, bacertia, and fungi.

The hypothesized fundamental mechanism of the effect used in the invention is that a laser (especially a UV laser) locally heating a substrate/gel is able to locally disrupt the crosslinking bonds in the substrate/gel. The stiffness of the substrate/gel is proportional to the number of crosslinkers per volume. By effectively decreasing the crosslinkers inside the substrate/gel, the latter becomes softer, i.e. more easily deformed. By preparing a substrate/gel with an initial number of crosslinkers (the preparation of such a gel is well known for the one skilled), the number of crosslinkers per volume can then be effectively locally reduced by focusing the laser beam, which strongly heats the gel, thus locally disrupting the crosslinkers and locally softening the gel. In a protein-based gel, the applied laser still locally heats the gel, but may induce more complex and varied effects which may include local denaturing or other changes leading to softening or stiffening of the gel.

The invention therefore allows a precise spatial modification of the elasticity of the substrate, e.g. by local laser-induced reduction of crosslinkers inside the substrate. This allows realizing spatial patterns with varying elasticity inside a three-dimensional gel which can be used for guiding the movement of cells grown on or in these substrates. By applying the laser energy, crosslinkers which give a gel its stiffness can be broken, which results in a softer material. The use of a focused laser beam gives precise spatial control of the elasticity, even spatial areas with a diameter of 1 µm or smaller can be precisely modified with respect to their elasticity.

One significant advantage of the present invention over existing technologies is the improvement of the spatial precision by a factor of approximately 100 to 1000: The method according to the invention allows a previously unrealized precision and control in preparing elastic substrates of varied elasticity. Previous techniques according to the state of the art have not been able to produce high-spatial resolution elasticity modifications, well controlled elastic changes and/or precise, sophisticated, and widely varied elastic patterns on very soft materials. Another of the essential advantages of the technique according to the invention is concurrently being able to precisely control the change in the stiffness, elastic modulus and/or elasticity by regulating the power and the time of laser exposure. For example by using no power or repeated full power, the stiffness can be left unchanged or can be effectively locally reduced to nearly 0.

The spatial and elastic precision provided by the present invention allows a previously unexplored size of substrate elasticity changes. This increase in precision allows individual cells or cell extensions to be guided along the generated elastic patterns, providing a previously inaccessible level of elastic patterning precision to cell culture studies.

Existing methods according to the state of the art, however, do not only not offer a spatial precision smaller than the scale of a cell nor well controlled elastic changes, but they also cannot be used flexibly with respect to cell behavior and cell positions: The inventive method, however, provides all these flexibilities. Spatially varying elastic patterns can be generated in response to the cell behavior and the cell positions: For example, one could begin with a plain elastic substrate containing cells and then in response to the cells behavior or position create elastic patterns to influence their behavior.

Therefore, a desired path for cell guidance can be created after the cells have been placed on the substrate (interactive control, this is not possible with the cell guidance techniques according to the state of the art).

The method according to the invention also offers the possibility of creating elastic patterns in two-dimensional space as well as in three-dimensional space: Due to the precise positioning possible with a focused laser (i.e. the precise positioning of the beam focus) not only an xy-control in one defined plane, but also a z-control perpendicular to that plane can be realized (the latter being a critical feature for potential in vivo applications).

The method can be used in vitro as well as in vivo: It has a wide variety of applications in biophysical sciences and biotechnology, for biomedical applications both in diagnostics and in therapeutic treatment (in vitro applications as well as in vivo applications).

The basis for this wide applicability is the fundamental importance of the manipulation of the substrate elasticity according to the invention: Cells are known to respond to different substrate elasticities. By locally changing the substrate elasticity, it is possible to locally change cell behavior and movement. In general, the ability to control cell movement and cell extension is of fundamental importance to a wide variety of applications in many diverse fields. For example elastically modified regions can work as excellent guidance paths for neuronal extension.

Additionally, elastic patterns realized with the method are a valuable technique to separate different cells. For example, in the metastatic transformation of normal tissue cells to more motile aggressive cancerous cells, it has been measured that the cells' elasticity also decreases (becomes softer). If cell spreading and movement are governed by the ratio of the substrate elasticity (which can be modified according to the invention) to cell elasticity, then softer cells are able to move on softer materials better than on stiffer cells. This can open a new technique to separate softer metastatic cells from stiffer normal cells, e.g. by using elastic gradients (i.e. gradient patterns).

When the laser is applied to gels, sometimes also a small amount is locally swelled vertically (approximately 3 µm). This is an additional effect not intended, but not a problem within the application of the method. However, in case of UV radiation, if the gel has particle contaminants or air bubbles, these may strongly absorb the UV radiation, causing larger heating and larger (approximately 20 µm), uncontrolled disruptions in the gel. Therefore, especially in the case of using UV radiation, a careful gel preparation is important.

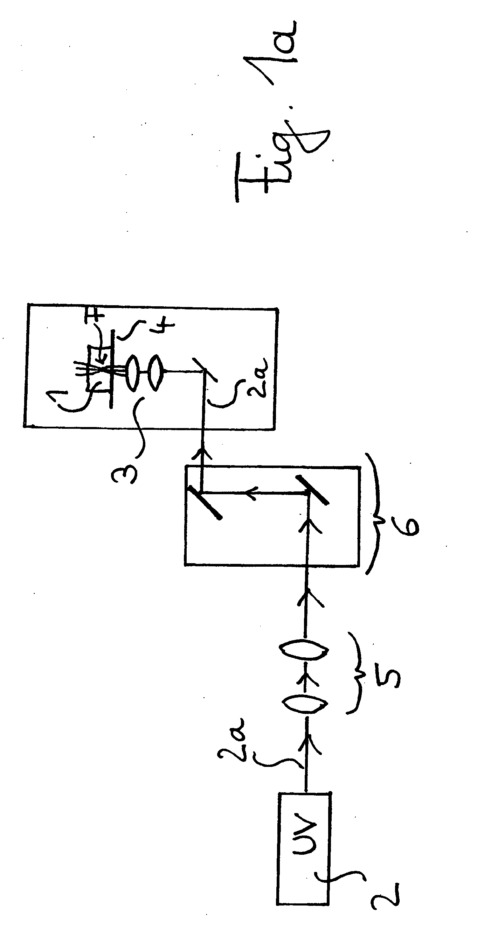

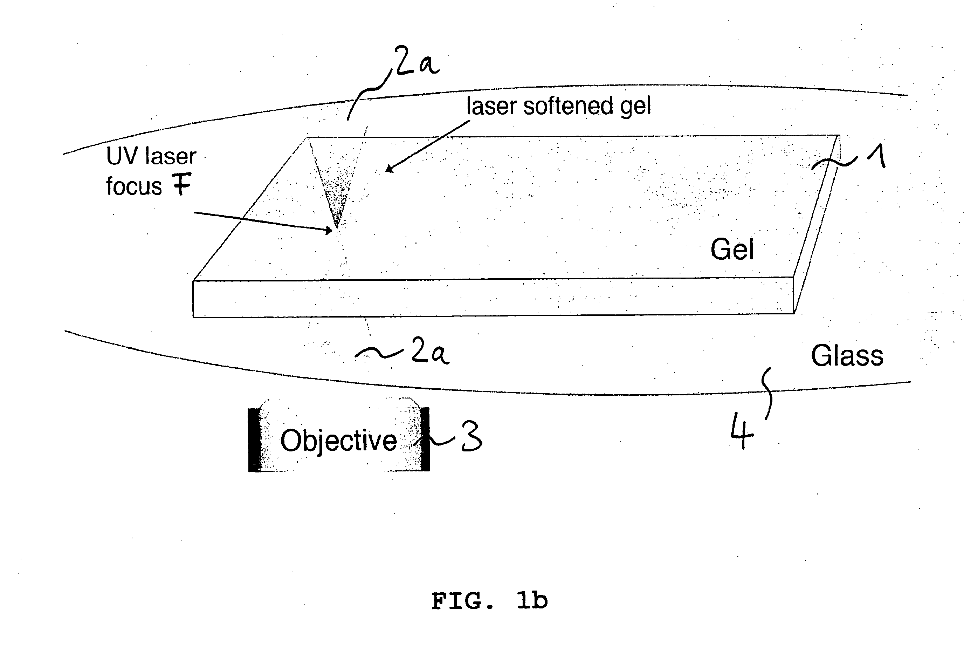

A specific embodiment of the method according to the invention is now described:
An exemplary optical modification method according to the invention is illustrated in figure 1a and figure 1b. The laser beam 2a of a UV laser 2 is directed to a telescope 5 for controlling the beam size to an appropriate beam diameter. The arrangement and the adjustment of such a telescope 5 is well known for the one skilled in the art; this holds also for the subsequent elements 6 and 3, which are therefore not further described in detail in the present invention. The beam 2a formed by the telescope 5 is directed to scanning microscope mirrors 6 in order to couple the laser beam into the objective 3 of a conventional inverted fluorescence microscope.

Positioned on a glass plate 4 in the focus range of the microscope, which is fixedly arranged on a stage (not shown) allowing the glass plate to be positioned relative to the focus F of the laser beam 2a (the stage may be coupled to a motor or similar device to position the glass plate 4 accordingly), the substrate 1, here a polyacrylamide gel, is arranged. The optical elements 2, 5, 6, 3 and the glass plate 4 are arranged and positioned such that the laser beam 2a of the UV laser 2 is focused inside the three-dimensional substrate 1 (figure 1b). By rotating the mirror elements arranged in front of the objective 3 accordingly, the focus F of the laser beam 2a has been moved through the substrate 1 (see line in figure 1b indicating a laser softened gel trace). The laser softened gel trace shown in figure 1b has been realized as a gradient trace, i.e. while moving the focus F through the substrate 1, the intensity of the laser beam has been gradually increased.

In the present case, a pulsed 337 nM UV laser has been used which can create small scale (smaller than 2 µm) inhomogenities since the laser is not constantly on while scanning the gel, but being pulsed at about 5 to 30 Hz depositing between 100 and 300 µJ per pulse. However, also a continuous wave laser can be used, which then creates smoother or more continuous patterns.

Instead of a UV laser, for example also a continuous wave infrared laser with a wavelength between 750 and 1064 nm can be used (e.g. a Ti:sapphire laser).

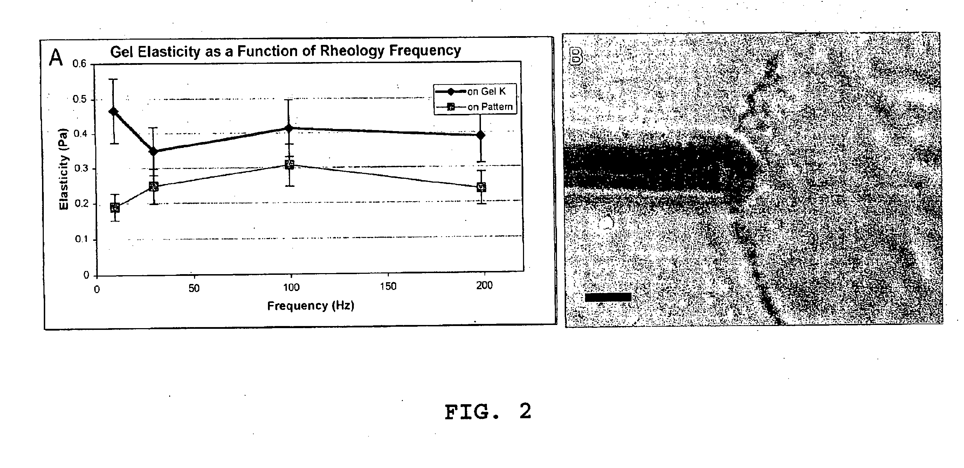

In figure 1b it can be seen that the gel 1 is adhered to a glass cover slip 4 which is placed in the microscope stage. The microscope objective 3 focuses the laser radiation 2a onto a precisely defined point F (focus) in three-dimensional space, where heating causes the gel crosslinkers to break and locally softens the gel. This softening can be seen in the phase contrast microscopy as a darkening of the gel (see figures 2 to 5) .

In this embodiment, an inverted microscope is used, where the microscope objective 3 is below the sample 1. The laser beam is emitted from the objective and precisely focused on the polyacrylamide gel, heating the gel only at the laser focus and not in adjacent regions so that the above mentioned precise heating and softening of the gel (while simultaneously darkening the gel, see figures) occurs. The amount necessary for softening the gel in a defined manner is readily controlled by the applied laser intensity and/or by the applied exposure time.

While a simple line pattern is illustrated in figure 1b, any spatial pattern may be generated with freedom both in the spatial distribution and elasticity reduction of the gel.

Instead of using a polyacrylamide gel 1, also gels such as agarose (Balgude et al, Biomaterials, 22, 2001), polymers, meltable materials, synthetic or natural extra-cellular matrix type material gels, or fibrin/thrombin (Ju et al., Biomaterials, 28, 2007) can be used.

It is therefore possible to use an extremely wide variety of two or three-dimensional gels within or on which cells may move.

It is also possible to include additional absorbing components in the gel. This is especially advantageous when continuous emission infrared or visible wavelength lasers are used: Normally infrared wavelengths are not highly absorbed by aqueous solutions, such as gels, so that the energy deposit, which can be realized with infrared lasers might be not sufficient. If therefore additional compounds, such as for example microscopic dust particles or the like or specific fluorophores, such as rhodamines or quantum dots, are introduced into the gel, it is possible to radically increase the absorption efficiency of the infrared laser light and to induce a significantly higher local heating.

Figure 2 shows a measured laser-induced reduction in gel elasticity according to the above described example. In panel A, the gel and the pattern elasticities are shown using atomic force microscopy (AFM) rheology. By comparing the difference between the bulk gel elasticity and pattern elasticity, one can see that the realized patterns are softer than the bulk gel (patterns: lower elasticity curve, bulk: upper curve). In panel B, the measured area is shown in phase contrast with the AFM cantilever over the patterned area (scale bar is 10 µm).

Figures 3 to 5 show further generated patterns in phase contrast microscopy images. Here, primary embryonic rat cortical neurons were grown on the patterned substrate and are shown to turn preferentially towards and grow along the softer patterned areas.

Figure 3 shows on overview of neuronal growth on elastic patterns. In this figure, one can see the generated elastic patterns as darker structures in the left panel at time zero. In the right panel, one can see that after 90 hrs. neurons which came in contact with the generated patterns followed these patterns. Several regions of interest marked as A, B and C are shown at higher magnification in figures 4 and 5. Thus, the neurons were observed to turn towards and extend preferentially along the softer regions. The growth of the neurons was continuously observed and recorded while they were cultured on the microscope stage in a pH and temperature controlled chamber well known for one skilled.

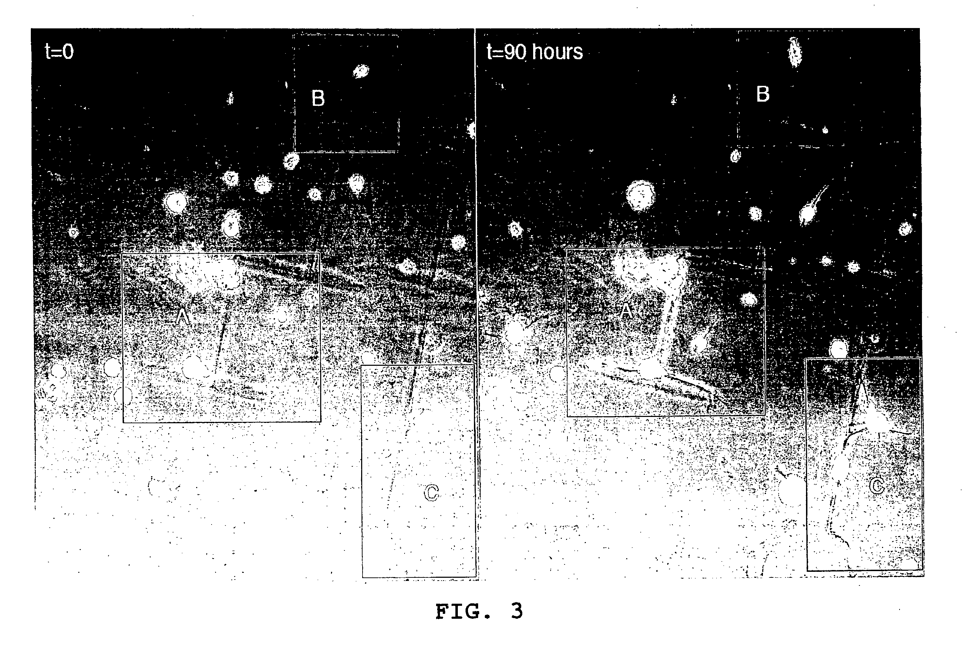

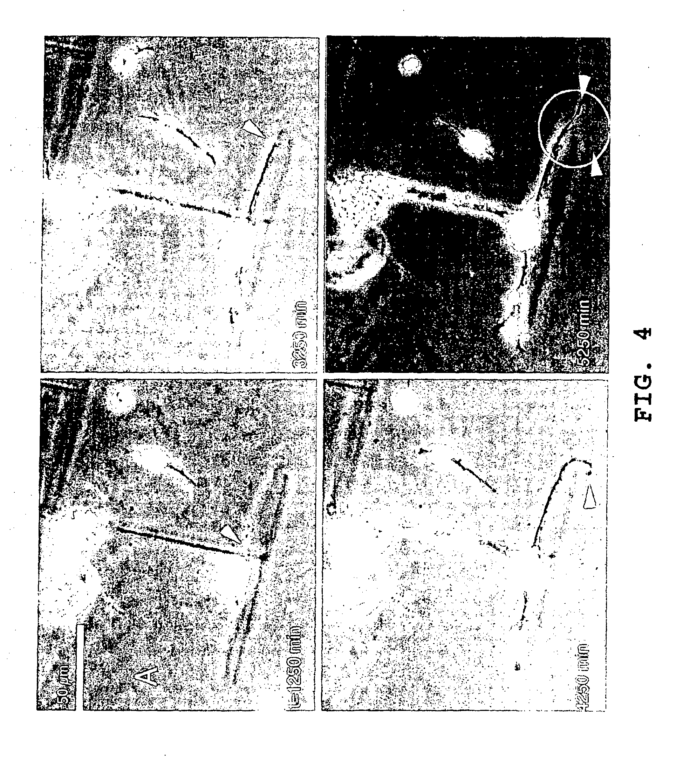

In figure 4 the inset A of figure 3 is shown and the migration of neurites (extensions of neurons) can be seen over time. The neurites extended along the softer patterned regions and when they reach the end of an elastic pattern, they were observed to turn approximately 180° to continue migrating along the pattern (see white arrows). In the bottom panels at times 4250 and 5250 mins., when the neurite reached the end of the pattern, it split in two where half of the neurite turned nearly 180° in order to remain the pattern, demonstrating the strength of the elastic pattern as a guidance technique.

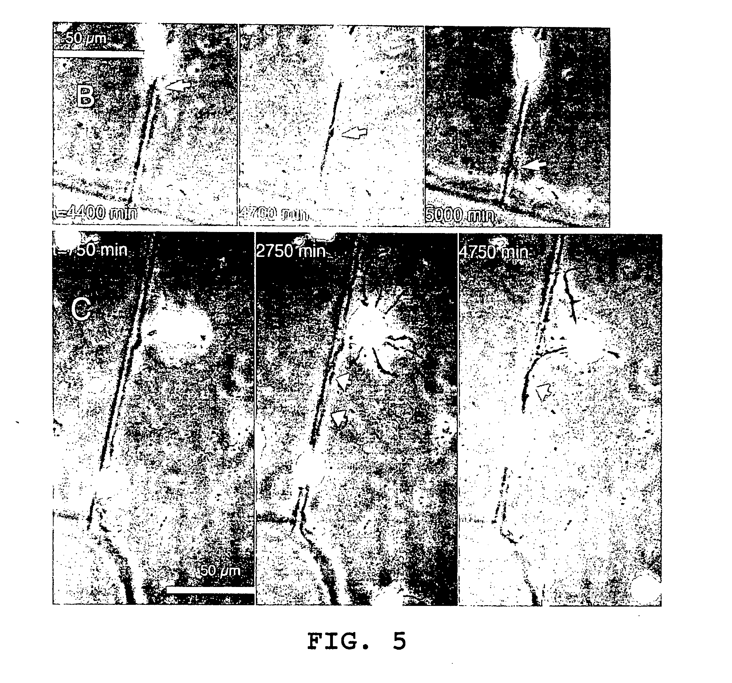

In figure 5, neurons are again observed to move along the elastic pattern, however, in this instance, two neurons on the same elastic path migrate towards each other, resulting in an intercellular contact. In the top panels of inset B, one can see the directed migration of a neurite along the elastic pattern, as indicated by the white arrows. In the bottom panels of inset C, one can see the migration of two neurites towards each other along an elastic pattern as indicated by the white arrows. This demonstrates the feasibility of connecting separate cells by generating an elastic pattern between them.

The process can be used to guide cells, particularly neurons, together to form intercellular connections, such as a synapse. Therefore, the elastic patterns realized according to the invention can be used as cell guidance patterns as well as cell connection patterns.

## Claims

1. Method for locally modifying the elasticity of an elastic substrate (1) designed for the growth of living cells thereon and/or therein, comprising:
emitting laser radiation (2a) by a laser (2),
locally depositing energy in at least one spatial area located inside and/or on the surface of the substrate (1) by focusing (F) the emitted laser radiation (2a) into/onto the at least one spatial area, wherein the amount of locally deposited energy is sufficient to locally modify the elasticity, stiffness and/or the elastic modulus of the substrate (1), and
populating, before, during or after the local energy deposition therein, the at least one predetermined spatial area in which the elasticity of the substrate (1) has been modified and/or at least one adjacent area of the substrate (1) at least partly with the cells.

2. Method according to the preceding claim, wherein the laser radiation (2a) is focused onto the predetermined spatial area by using a lens system, preferably a microscope objective, preferably the objective of an inverted microscope.

3. Method according to one of the preceding claims, wherein the laser (2) is a UV laser or an infrared laser, preferably a Ti:Sapphire laser and/or an infrared laser with a wavelength between 750 nm and 1064 nm,
and/or
wherein the laser (2) is a continuous wave laser or a pulsed laser, preferably a pulsed laser with a pulse frequency between 3 and 100 Hz, especially between 5 and 30 Hz.

4. Method according to one of the preceding claims, wherein the properties of the laser radiation, especially the locally deposited energy density, the average beam intensity, the time duration for irradiating the predetermined area, the pulse frequency of the laser and/or the beam waist of the laser radiation is selected thus that a predetermined percentage of crosslinkers, preferably between 10 and 50%, which give the substrate its stiffness are broken in order to change the elasticity of the substrate in at least one of the modified spatial areas.

5. Method according to one of the preceding claims, wherein a spatial pattern of locally varying elasticity, stiffness and/or the elastic modulus comprising multiple locally modified spatial areas is created inside and/or on the surface of the elastic substrate (1)
and/or
wherein at least one of the spatial areas comprises a dot, a straight line and/or a curved line.

6. Method according to the preceding claim, wherein the pattern comprises a gradient shaped variation of the elasticity, stiffness and/or the elastic modulus, i.e. a decrease or an increase of the elasticity of modified spatial areas along a direction inside the substrate (1).

7. Method according to one of the two preceding claims, wherein a path for cell movement or growth shaped in a predetermined form is created as the spatial pattern.

8. Method according to one of the preceding claims, wherein living cells are distributed and/or grown in the elastic substrate (1).

9. Method according to the preceding claim and according to claim 5, wherein the spatial pattern is generated in response to a behaviour, preferably a growth direction, of the distributed and/or grown cells which has been determined prior to the generation of said spatial pattern and/or in response to positions and/or spatial distributions of the distributed and/or grown cells which have been determined prior to the generation of said spatial pattern.

10. Method according to one of the preceding claims, wherein the substrate comprises a natural or synthetic gel, preferably a polyacrylamide gel or a porous gel such as but not limited to agarose, fibrin and/or thrombin, synthesized or natural material especially extra-cellular matrix material, or a collagen matrix,
and/or
wherein the living cells comprise eukaryotic cells, mammalian cells, human cells, animal cells, neurons and/or neuronal precursors.

11. Method according to one of the preceding claims, wherein a predetermined absorption component, especially particulate matter particles with a preferable particle size between 1 nm and 1 µm and/or particles with fluorophore components, adapted to absorb electromagnetic radiation in the wavelength range of the laser radiation (2a) is introduced into the substrate prior to irradiate it with the laser radiation (2a).

12. Method according to one of the preceding claims, wherein the laser radiation (2a) is focused with an average beam intensity in 1 mW, 100 mW, preferably in 10 mW, 50 mW, and/or with an exposure time in 1 ms, 500 ms, preferably in 1 ms, 50 ms, and/or with pulses of 1-1000 µJ, preferably 100-500 µJ onto at least one of the predetermined spatial areas.

13. Method according to one of the preceding claims, wherein at least one of the predetermined spatial areas has a size, length and/or diameter of between 200 nm to 100 µm, preferably between 500 nm and 10 µm.

14. Method according to one of the preceding claims, wherein an elastically modified substrate is used to separate a heterogeneous population of cells based on their responses to and ability to migrate on or within the elastically modified substrate.

15. Use of a method according to one of the preceding claims for guidance of living cells and/or for inducing a connection between multiple cells, preferably for defining at least one cell movement and/or growth path.

## Patentansprüche

1. Verfahren zum lokalen Modifizieren der Elastizität eines elastischen Substrats (1), das für das Wachstum lebender Zellen darauf und/oder darin gestaltet ist, aufweisend:
Emittieren von Laserstrahlung (2a) durch einen Laser (2),
lokales Einbringen von Energie in zumindest einen räumlichen Bereich, der innerhalb und/oder auf der Oberfläche des Substrats (1) verortet ist, durch Fokussieren (F) der emittierten Laserstrahlung (2a) in/auf den zumindest einen räumlichen Bereich, wobei der Betrag von örtlich eingebrachter Energie hinreichend ist, um lokal die Elastizität, Steifigkeit und/oder das Elastizitätsmodul des Substrats (1) zu modifizieren, und
Populieren, vor, während oder nach dem lokalen Einbringen der Energie darein, des zumindest einen vorbestimmten räumlichen Bereiches, in dem die Elastizität des Substrats (1) modifiziert wurde und/oder zumindest eines angrenzenden Bereiches des Substrats (1) zumindest teilweise mit Zellen.

2. Verfahren nach dem vorhergehenden Anspruch, wobei die Laserstrahlung (2a) unter Verwendung eines Linsensystems, vorzugsweise eines Mikroskop-Objektivs, vorzugsweise des Objektivs eines invertierten Mikroskops, auf den vorbestimmten räumlichen Bereich fokussiert wird.

3. Verfahren nach einem der vorhergehenden Ansprüche, wobei der Laser (2) ein UV-Laser oder ein Infrarot-Laser, vorzugsweise ein Ti: Sapphire-Laser und/oder ein Infrarot-Laser mit einer Wellenlänge zwischen 750 nm und 1064 nm ist; und/oder
wobei der Laser (2) ein Kontinuierliche-Wellen-Laser oder ein gepulster Laser, bevorzugt ein gepulster Laser mit einer Pulsfrequenz zwischen 3 und 100 Hz, insbesondere zwischen 5 und 30 Hz, ist.

4. Verfahren nach einem der vorhergehenden Ansprüche, wobei die Eigenschaften der Laserstrahlung, insbesondere die lokal eingebrachte Energiedichte, die durchschnittliche Strahlintensität, die Zeitdauer zum Bestrahlen des vorbestimmten Bereichs, die Pulsfrequenz des Lasers und/oder die Strahlbreite der Laserstrahlung so gewählt ist, dass ein vorbestimmter Prozentsatz von Verbindungen (cross linkers), vorzugsweise zwischen 10 und 50 %, die dem Substrat seine Steifigkeit geben, gebrochen werden, um die Elastizität des Substrats in zumindest einem der modifizierten räumlichen Bereiche zu ändern.

5. Verfahren nach einem der vorhergehenden Ansprüche, wobei ein räumliches Muster von lokal variierender Elastizität, Steifigkeit und/oder dem Elastizitätsmodul, das eine Vielzahl lokal modifizierter räumlicher Bereiche aufweist, im Inneren und/oder auf der Oberfläche des elastischen Substrats (1) geschaffen wird
und/oder
wobei zumindest einer der räumlichen Bereiche einen Punkt, eine gerade Linie und/oder eine gekrümmte Linie aufweist.

6. Verfahren nach dem vorhergehenden Anspruch, wobei das Muster eine gradientenförmige Variation der Elastizität, Steifigkeit und/oder des Elastizitätsmoduls aufweist, d. h. eine Abnahme oder eine Zunahme der Elastizität von modifizierten räumlichen Bereichen entlang einer Richtung innerhalb des Substrats (1).

7. Verfahren nach einem der zwei vorhergehenden Ansprüche, wobei ein Pfad für Zellbewegung oder -wachstum, der in einer vorgegebenen Form geformt ist, als das räumliche Muster erzeugt wird.

8. Verfahren nach einem der vorhergehenden Ansprüche, wobei lebende Zellen in dem elastischen Substrat (1) verteilt und/oder gewachsen werden.

9. Verfahren nach dem vorhergehenden Anspruch und nach Anspruch 5, wobei das räumliche Muster in Reaktion auf ein Verhalten, vorzugsweise eine Wachstumsrichtung, der verteilten und/oder gewachsenen Zellen, erzeugt wird, das vor der Erzeugung des besagten räumlichen Musters und/oder in Reaktion auf Positionen und/oder räumliche Verteilungen der verteilten und/oder gewachsenen Zellen, die vor der Erzeugung des besagten räumlichen Musters bestimmt worden sind.

10. Verfahren nach einem der vorhergehenden Ansprüche, wobei das Substrat ein natürliches oder synthetisches Gel, vorzugsweise ein Polyacrylamid-Gel oder ein poröses Gel, wie z. B. aber nicht beschränkt auf Agarose, Fibrin und/oder Thrombin, synthetisiertes oder natürliches Material, insbesondere extra-zelluläres Matrix-Material oder eine Kollagen-Matrix enthält,
und/oder
wobei die lebenden Zellen eukariotische Zellen, Säugetierzellen, menschliche Zellen, Tierzellen, Neuronen und/oder neuronale Precurser aufweisen.

11. Verfahren nach einem der vorhergehenden Ansprüche, wobei eine vorbestimmte Absorptionskomponente, insbesondere Staubteilchen mit einer bevorzugten Teilchengröße zwischen 1 nm und 1 µm und/oder Teilchen mit Luminophor (Fluorophor)-Komponenten, die angepasst sind, elektromagnetische Strahlung im Wellenlängenbereich der Laserstrahlung (2a) zu absorbieren, in das Substrat vor seiner Bestrahlung mit der Laserstrahlung (2a) eingebracht wird.

12. Verfahren nach einem der vorhergehenden Ansprüche, wobei die Laserstrahlung (2a) mit einer durchschnittlichen Strahlintensität zwischen 1 mW und 100 mW, vorzugsweise zwischen 10 mW und 50 mW und/oder mit einer Belichtungszeit zwischen 1 ms und 500 ms, vorzugsweise zwischen 1 ms und 50 ms und/oder mit Pulsen von 1 bis 1000 µJ, vorzugsweise 100 bis 500 µJ auf zumindest der einen der vorbestimmten räumlichen Bereiche fokussiert wird.

13. Verfahren nach einem der vorhergehenden Ansprüche, wobei mindestens einer der vorbestimmten räumlichen Bereiche eine Größe, Länge und/oder Durchmesser von zwischen 200 nm bis 100 µm, vorzugsweise zwischen 500 nm und 10 µm hat.

14. Verfahren nach einem der vorhergehenden Ansprüche, wobei ein elastisch modifiziertes Substrat verwendet wird, um eine heterogene Population von Zellen basierend auf Ihren Antworten auf und Fähigkeit zu migrieren auf oder innerhalb des elastisch modifizierten Substrats zu separieren.

15. Verwendung eines Verfahrens nach einem der vorhergehenden Ansprüche zum Leiten von lebenden Zellen und/oder zum Einbringen einer Verbindung zwischen eine Vielzahl von Zellen, vorzugsweise zum Definieren mindestens einer Zellbewegung und/oder eines Wachstumspfades.

## Revendications

1. Procédé pour modifier localement l'élasticité d'un substrat élastique (1) conçu pour la croissance de cellules vivantes sur celui-ci et/ou dans celui-ci, comprenant :
l'émission d'un rayonnement laser (2a) par un laser (2),
le dépôt localement d'énergie dans au moins une zone spatiale située à l'intérieur et/ou sur la surface du substrat (1) en focalisant (F) le rayonnement laser (2a) émis dans/sur ladite au moins une zone spatiale, dans lequel la quantité d'énergie déposée localement est suffisante pour modifier localement l'élasticité, la rigidité et/ou le module d'élasticité du substrat (1), et
le peuplement, avant, pendant ou après le dépôt d'énergie localement dans celle-ci, de ladite au moins une zone spatiale prédéterminée dans laquelle l'élasticité du substrat (1) a été modifiée et/ou d'au moins une zone adjacente du substrat (1) au moins partiellement avec les cellules.

2. Procédé selon la revendication précédente, dans lequel le rayonnement laser (2a) est focalisé sur la zone spatiale prédéterminée en utilisant un système de lentilles, de préférence un objectif de microscope, de préférence l'objectif d'un microscope inversé.

3. Procédé selon l'une des revendications précédentes, dans lequel le laser (2) est un laser UV ou un laser infrarouge, de préférence un laser Ti:saphire et/ou un laser infrarouge avec une longueur d'onde entre 750 nm et 1 064 nm,
et/ou
dans lequel le laser (2) est un laser à onde continue ou un laser pulsé, de préférence un laser pulsé avec une fréquence d'impulsion entre 3 et 100 Hz, en particulier entre 5 et 30 Hz.

4. Procédé selon l'une des revendications précédentes, dans lequel les propriétés du rayonnement laser, en particulier la densité d'énergie déposée localement, l'intensité de faisceau moyenne, la durée d'irradiation de la zone prédéterminée, la fréquence d'impulsion du laser et/ou la taille de faisceau du rayonnement laser sont sélectionnées de sorte qu'un pourcentage prédéterminé d'agents de réticulation, de préférence entre 10 et 50 %, qui donnent au substrat sa rigidité soient brisés afin de changer l'élasticité du substrat dans au moins l'une des zones spatiales modifiées.

5. Procédé selon l'une des revendications précédentes, dans lequel un motif spatial d'élasticité, de rigidité et/ou du module d'élasticité variant localement comprenant de multiples zones spatiales modifiées localement est créé à l'intérieur et/ou sur la surface du substrat élastique (1)
et/ou
dans lequel au moins l'une des zones spatiales comprend un point, une droite et/ou une ligne incurvée.

6. Procédé selon la revendication précédente, dans lequel le motif comprend une variation en forme de gradient de l'élasticité, de la rigidité et/ou du module d'élasticité, c'est-à-dire une diminution ou une augmentation de l'élasticité des zones spatiales modifiées le long d'une direction à l'intérieur du substrat (1).

7. Procédé selon l'une des deux revendications précédentes, dans lequel un trajet pour le déplacement ou la croissance de cellules formé en une forme prédéterminée est créé en tant que motif spatial.

8. Procédé selon l'une des revendications précédentes, dans lequel des cellules vivantes sont réparties et/ou développées dans le substrat élastique (1).

9. Procédé selon la revendication précédente et selon la revendication 5, dans lequel le motif spatial est généré en réponse à un comportement, de préférence une direction de croissance, des cellules réparties et/ou développées qui a été déterminé avant la génération dudit motif spatial et/ou en réponse aux positions et/ou aux répartitions spatiales des cellules réparties et/ou développées qui ont été déterminées avant la génération dudit motif spatial.

10. Procédé selon l'une des revendications précédentes, dans lequel le substrat comprend un gel naturel ou synthétique, de préférence un gel de polyacrylamide ou un gel poreux, tel que, mais sans y être limité, de l'agarose, de la fibrine et/ou de la thrombine, un matériau synthétisé ou naturel en particulier un matériau matriciel extracellulaire, ou une matrice de collagène
et/ou
dans lequel les cellules vivantes comprennent des cellules eucaryotes, des cellules de mammifères, des cellules humaines, des cellules animales, des neurones et/ou des précurseurs neuronaux.

11. Procédé selon l'une des revendications précédentes, dans lequel un composant d'absorption prédéterminé, en particulier des particules de substance particulaire avec une taille de particule préférable entre 1 nm et 1 µm et/ou des particules avec des composants de fluorophore, conçu pour absorber un rayonnement électromagnétique dans la plage de longueur d'onde du rayonnement laser (2a) est introduit dans le substrat avant qu'il soit irradié avec le rayonnement laser (2a).

12. Procédé selon l'une des revendications précédentes, dans lequel le rayonnement laser (2a) est focalisé avec une intensité de faisceau moyenne de 1 mW, 100 mW, de préférence de 10 mW, 50 mW, et/ou avec un temps d'exposition de 1 ms, 500 ms, de préférence de 1 ms, 50 ms, et/ou avec des impulsions de 1 à 1 000 µJ, de préférence 100 à 500 µJ sur au moins l'une des zones spatiales prédéterminées.

13. Procédé selon l'une des revendications précédentes, dans lequel au moins l'une des zones spatiales prédéterminées a une taille, une longueur et/ou un diamètre entre 200 nm et 100 µm, de préférence entre 500 nm et 10 µm.

14. Procédé selon l'une des revendications précédentes, dans lequel un substrat modifié élastiquement est utilisé pour séparer une population hétérogène de cellules sur la base de leurs réponses et de leur capacité à migrer sur ou dans le substrat modifié de manière élastique.

15. Utilisation d'un procédé selon l'une des revendications précédentes pour guider des cellules vivantes et/ou pour induire une liaison entre de multiples cellules, de préférence pour définir au moins un trajet de déplacement et/ou de croissance de cellules.
